(19) Europäisches Patentamt
European Patent Office
Office européen des brevets

(11) **EP 1 674 035 B1**

(12) **FASCICULE DE BREVET EUROPEEN**

(45) Date de publication et mention
de la délivrance du brevet:
**02.12.2009 Bulletin 2009/49**

(51) Int Cl.:
***A61B 5/02*** *(2006.01)*

(21) Numéro de dépôt: **05292668.0**

(22) Date de dépôt: **14.12.2005**

(54) **Appareillage pour le diagnostic non invasif des états de syncope vasovagale chez un patient**

Diagnosevorrichtung zur nicht-invasiven Diagnose von Zuständen eines vasovagalen Kollapses bei einem Patienten

Diagnostic apparatus for non invasive diagnostic of vasovagal syncope states of a patient

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **21.12.2004 FR 0413646**

(43) Date de publication de la demande:
**28.06.2006 Bulletin 2006/26**

(73) Titulaire: **ELA MEDICAL**
**92541 Montrouge (FR)**

(72) Inventeurs:
• **Vitali, Luca**
**10019 Strambino (TO) (IT)**
• **Gaggini, Guido**
**20128 Milano (IT)**

(74) Mandataire: **Dupuis-Latour, Dominique et al**
**Bardehle Pagenberg Dost Altenburg Geissler**
**10, boulevard Haussmann**
**75009 Paris (FR)**

(56) Documents cités:
**US-A- 5 957 957**      **US-A- 6 044 297**
**US-A1- 2003 040 776**      **US-A1- 2004 215 263**

**Description**

L'invention concerne un appareillage pour le diagnostic non invasif des états de syncope vasovagale chez un patient.

Essentiellement, une syncope est une perte de connaissance temporaire avec chute du tonus musculaire, résultant de la réduction momentanée de la circulation cérébrale. Parmi différents types de syncopes, la syncope vasovagale est celle provoquée par un déséquilibre temporaire du système de régulation de l'équilibre vasovagal, conduisant à une activation excessive du système vagal se traduisant par une vasodilatation et une bradycardie conduisant à la syncope.

On considère généralement que la syncope vasovagale provient d'un état où le système sympathique présente une réactivité particulièrement élevée qui a pour effet de déclencher une réponse antagoniste, excessive, du système parasympathique provoquant une vasodilatation induisant elle-même la réduction du remplissage des ventricules et la bradycardie.

La complexité de ces mécanismes rend malaisée l'étiologie de la syncope vasovagale, avec par voie de conséquence la difficulté à prescrire une thérapie appropriée, pharmacologique ou non.

Jusqu'à présent, il n'existait que des méthodes complexes et invasives pour l'étude du comportement du système nerveux autonome d'un sujet, donc de mise en oeuvre limitée et généralement restreinte à quelques cas particuliers.

Le US 2004/0215263 décrit un dispositif permettant d'anticiper la survenue d'une syncope vasovagale, en accord avec certains termes du préambul de la revendication 1, (VVS) pour déclencher une thérapie préventive par une commande appropriée d'un stimulateur cardiaque. Le dispositif tient compte de la posture du patient ainsi que de divers signaux physiologiques tels que le rythme cardiaque et la pression sanguine.

Le US 5 957957 décrit plus en détail la manière d'évaluer une probabilité d'apparition d'une VVS en fonction de données de posture et d'activité du patient produites par un accéléromètre intégré au stimulateur cardiaque.

Il a également été proposé par Mangin et coll., "Simultaneous Analysis of Heart Rate Variability and Myocardial Contractility During Head-Up Tilt in Patients with Vasovagal Syncope", Journal of Cardiovascular Electro-physiology, Vol. 12, 6, 639-644, June 2001, d'utiliser un signal d'accélération endocardiaque délivré par une prothèse implantée pour analyser l'activité sympathico-vagale d'un patient.

Il existe en effet, comme cela est par exemple décrit dans le EP-A-0 515 319 (Sorin Biomedica Cardio SpA), des stimulateurs associés à une sonde endocavitaire dont l'électrode distale implantée au fond du ventricule intègre un micro-accéléromètre permettant de mesurer l'accélération endocardiaque. Le EP-A-0 655 260 (Sorin Biomedica Cardio SpA) décrit une manière de traiter le signal d'accélération endocavitaire délivré par ce capteur situé en bout de sonde pour détecter certains troubles cardiaques et déclencher éventuellement une thérapie de défibrillation.

L'article précité décrit le résultat d'études cliniques effectuées sur une série de patients appareillés de telles prothèses, chez lesquels on tente de provoquer une syncope par une épreuve d'inclinaison ("tilt-test"), qui est une épreuve en elle-même bien connue dont le but est de mettre en évidence l'origine d'une syncope susceptible d'être provoquée lorsqu'un patient installé sur une table basculante passe d'une position couchée à une position fortement inclinée, le résultat étant considéré comme positif en cas d'apparition dans un temps donné des symptômes fonctionnels d'une syncope, avec une hypotension sévère éventuellement associée à une bradycardie paradoxale.

L'étude rapportée dans cet article met en lumière une corrélation entre les niveaux des pics d'accélération endocardiaque (PEA) et la propension à développer une syncope.

L'étude reconnaît cependant la limitation due au faible nombre de patients, qui sont nécessairement des patients appareillés ayant reçu un stimulateur cardiaque spécialement pourvu de moyens de mesure de l'accélération endocardiaque.

De plus, elle ne tire aucune conséquence, ni sur le plan thérapeutique ni sur le plan diagnostique, de la corrélation trouvée entre les variations des PEA et la survenue d'une syncope vasovagale.

L'un des buts de la présente invention est de mettre à la disposition des praticiens un appareillage pour le diagnostic des états de syncope vasovagale qui, d'une part, soit non invasif (et donc applicable à tout patient, qu'il soit ou non appareillé d'un stimulateur) et qui, d'autre part, délivre au praticien non seulement des données brutes (variations des PEA, fréquence cardiaque) mais également assure une aide au diagnostic, notamment sur le type de syncope développée par le patient.

L'appareillage de l'invention est du type général décrit dans l'article de Mangin et coll., c'est-à-dire comportant des moyens de recueil de l'accélération endocardiaque du patient, des moyens de recueil de la fréquence cardiaque du patient, et des moyens d'analyse, recevant en entrée ladite accélération endocardiaque et ladite fréquence cardiaque et délivrant en sortie une information sur l'activité sympathico-vagale du patient.

De façon caractéristique de l'invention, les moyens de recueil de l'accélération endocardiaque comprennent un capteur accélérométrique externe apte à être maintenu en contact avec la paroi thoracique du patient, et les moyens d'analyse comprennent des moyens classificateurs aptes, en cas de survenue d'une syncope, à déterminer un type de syncope parmi plusieurs, en fonction des valeurs de l'accélération endocardiaque et de la fréquence cardiaque recueillies au cours d'une pluralité de cycles cardiaques précédant la survenue de la syncope.

Très avantageusement, les moyens d'analyse déterminent au moins une valeur fonction de l'un et/ou de l'autre de deux pics de l'accélération endocardiaque au cours d'un cycle donné, ces deux pics comprenant un premier pic lors de la phase de contraction ventriculaire isovolumique et un second pic lors de la phase de relaxation ventriculaire isovolumique. Les moyens classificateurs déterminent alors le type de syncope en fonction de valeurs de pic(s) d'accélération endocardiaque et de la fréquence cardiaque recueillies au cours d'une pluralité de cycles cardiaques précédant la survenue de la syncope.

Les moyens classificateurs peuvent notamment déterminer le type de syncope en fonction d'une moyenne, et/ou de la variation, et/ou d'une différence entre une moyenne à long terme et une moyenne à court terme, des valeurs de pic(s) d'accélération endocardiaque recueillies au cours d'une pluralité de cycles cardiaques précédant la survenue de la syncope.

Tout particulièrement, les moyens d'analyse déterminent des moyennes à long terme à court terme des valeurs des premiers et seconds pics d'accélération endocardiaque et des valeurs de fréquence cardiaque, les moyens classificateurs déterminant alors le type de syncope en fonction de différences entre ces moyennes à long terme et à court terme recueillies au cours d'une pluralité de cycles cardiaques précédant la survenue de la syncope.

Un premier type de syncope peut ainsi être déterminé lorsque, à un premier facteur de proportionnalité près, la moyenne à court terme des valeurs des premiers pics d'accélération est inférieure à la moyenne à long terme des valeurs des premiers pics d'accélération et, à un deuxième facteur de proportionnalité près, la moyenne à court terme des valeurs de fréquence cardiaque est supérieure à la moyenne à long terme des valeurs de fréquence cardiaque.

Un deuxième type de syncope peut ainsi être déterminé lorsque, à un troitroisième facteur de proportionnalité près, la moyenne à court terme des valeurs des seconds pics d'accélération est inférieure à la moyenne à long terme des valeurs des seconds pics d'accélération et, à un quatrième facteur de proportionnalité près,la moyenne à court terme des valeurs de fréquence cardiaque est supérieure à la moyenne à long terme des valeurs de fréquence cardiaque.

Un troisième type de syncope peut ainsi être déterminé lorsque, à un cinquième facteur de proportionnalité près, inférieur audit troisième facteur de proportionnalité, la moyenne à court terme des valeurs des seconds pics d'accélération est inférieure à la moyenne à long terme des valeurs des seconds pics d'accélération et, à un sixième facteur de proportionnalité près, inférieur audit quatrième facteur de proportionnalité, la moyenne à court terme des valeurs de fréquence cardiaque est supérieure à la moyenne à long terme des valeurs de fréquence cardiaque.

On va maintenant décrire un exemple de réalisation de l'invention, en référence aux dessins annexés.

La figure 1 est une vue schématique de l'appareillage de l'invention, relié à un patient installé sur une table basculante.
La figure 2 précise la structure interne du capteur accélérométrique utilisé.
La figure 3 est un schéma des circuits d'entrée et de mise en forme du signal délivré par le capteur de la figure 3.
La figure 4 est un chronogramme montrant les variations de l'accélération endocavitaire ainsi que de l'électrogramme et de l'électrocardiogramme de surface correspondants, au cours de trois cycles cardiaques successifs.

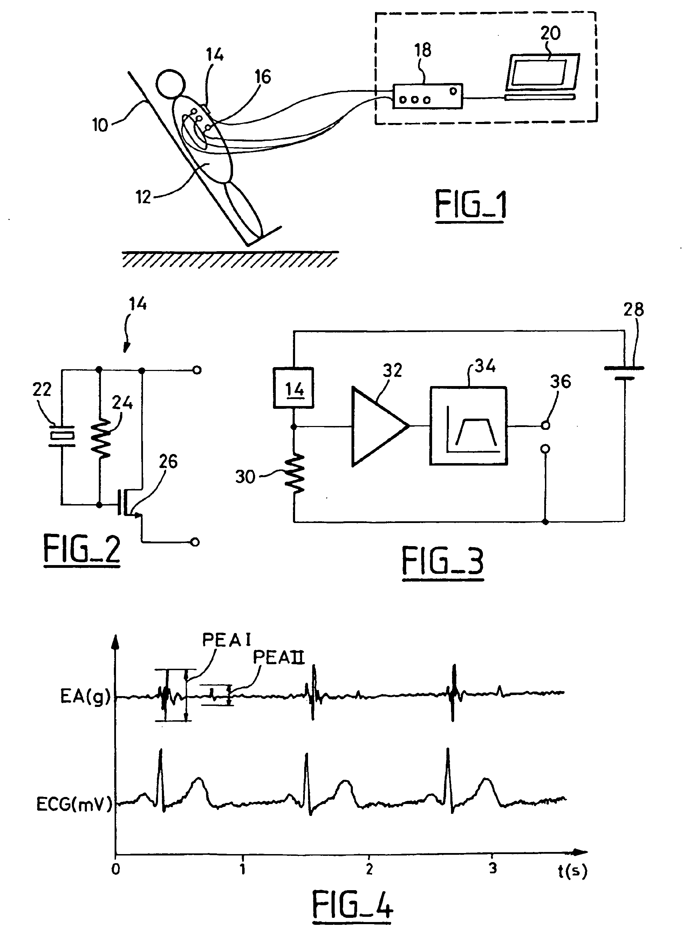

Sur la figure 1, la référence 10 désigne une table basculante sur laquelle est installé un patient 12 devant subir une épreuve d'inclinaison, conformément à une pratique en elle-même classique dans le domaine du diagnostic des syncopes.

De façon caractéristique de l'invention, le patient est équipé d'un capteur accélérométrique externe 14 placé dans la région du sternum et maintenu plaqué contre la paroi thoracique, par exemple (mais de manière non limitative) par un "patch" tel que celui utilisé pour le maintien des électrodes ECG, le tout étant éventuellement recouvert d'un bandage adhésif.

Le patient est également équipé d'électrodes 16 de recueil d'un ECG de surface, les signaux recueillis par le capteur 14 et les électrodes 16 étant appliqués à un équipement 18 d'amplification et de traitement des signaux délivrant des informations numérisées à un organe de traitement et d'affichage 20 tel qu'un micro-ordinateur permettant de présenter et d'analyser en temps réel les signaux délivrés par le capteur 14 et les électrodes 16.

La figure 2 montre plus en détail la structure du capteur accélérométrique 14, qui comporte un élément sensible piézoélectrique 22 polarisé par une résistance 24 et associé à un transistor MOS 26 de préamplification.

Comme on peut le voir sur la figure 3, le capteur 14 est polarisé par une source de tension 28 en série avec une résistance 30. Le signal de sortie du capteur est amplifié par un amplificateur 32 puis mis en forme par un filtre passe-bande 34 et délivré à la sortie 36 pour numérisation et traitement ultérieurs.

Sur la figure 4, on a représenté (courbe du haut), les variations de l'accélération endocardiaque (EA) mesurée par le capteur 14. On a également illustré sur cette figure le tracé d'un électrocardiogramme de surface (ECG) correspondant, au cours de trois cycles cardiaques consécutifs.

Comme on peut le voir, l'accélération endocardiaque passe par deux pics successifs, dont l'amplitude peut être déterminée par un traitement approprié du signal délivré par le capteur d'accélération, comme décrit dans le EP-A-0 655 260 précité. Par "pic" on entendra la valeur maximale crête-à-crête du signal d'accélération séparant les deux extrema, positif et négatif, correspondant aux écarts PEA I et PEA II indiqués sur le chronogramme de la figure 4.

Plus précisément, le premier pic d'accélération endocardiaque ("PEA I") correspond à la fermeture des valvules mitrale et tricuspide, au début de la phase de contraction ventriculaire isovolumique (systole). Les variations de ce premier pic sont étroitement liés aux variations de la pression dans le ventricule (l'amplitude du pic PEA I étant, plus précisément, corrélée au maximum positif de la variation de pression dP/dt dans le ventricule gauche) et peuvent donc constituer un paramètre représentatif de la contractilité du myocarde, elle-même liée au niveau d'activité du système sympathique.

Le second pic d'accélération endocardiaque ("PEA II"), quant à lui, correspond à la fermeture des valvules aortique et pulmonaire, au moment de la phase de relaxation ventriculaire isovolumique. Ce second pic, qui est produit par la décélération brusque de la masse sanguine en mouvement dans l'aorte, constitue un paramètre représentatif de la pression sanguine périphérique au début de la diastole. Il constitue également un paramètre-clé du processus physiologique conduisant à la survenue d'une syncope vasovagale.

Les études cliniques ont montré que lors d'une épreuve d'inclinaison, les variations du PEA I, du PEA II et de la fréquence cardiaque évoluent suivant des schémas caractéristiques, qui peuvent être utilisés pour opérer une différenciation entre différents types d'instabilités du système nerveux autonome, et constituer ainsi une aide au diagnostic et donc à la définition d'une thérapie et du suivi de chaque patient.

L'épreuve d'inclinaison peut être réalisée en suivant le protocole classique dit "protocole Westminster" : au début, le patient repose en décubitus dorsal sur la table horizontale pendant 30 minutes, puis la table est relevée à 60° et le patient reste dans cette position pendant une durée maximale de 45 minutes, après quoi la table est replacée à l'horizontale. Si une syncope survient avant la fin de la période d'inclinaison, l'épreuve est considérée comme positive, sinon elle est considérée comme négative (absence de syncope).

L'accélération endocardiaque et la fréquence cardiaque sont suivies pendant toute la durée de cette épreuve, et l'appareillage détermine en temps réel les valeurs des paramètres PEA 1, PEA II et de la fréquence cardiaque.

Ces valeurs sont relevées au cours de cycles successifs et analysées pour opérer une classification de la syncope vasovagale. On notera que l'analyse peut être appliquée au signal PEA I seul, au signal PEA II seul, ou bien à une combinaison des deux signaux PEA I et PEA II associés à la fréquence cardiaque.

Il est possible de déterminer, cycle à cycle, les valeurs absolues que prenprennent ces paramètres par rapport à des seuils - ou, de préférence, en déterminer une valeur moyennée sur un nombre prédéterminé de cycles pour éviter les influences de la variabilité cycle-à-cycle (dispersion des mesures) et celles des évènements brefs non significatifs.

Pour améliorer la spécificité de la classification, et notamment pour tenir compte des différences de valeur de base des paramètres PEA d'un individu à l'autre, il peut être avantageux d'analyser les variations de ces paramètres plutôt que leurs valeurs absolues.

Une manière de procéder consiste à analyser la différence entre une moyenne à court terme et une moyenne à long terme du même paramètre. Si ce paramètre varie peu, la différence sera faible et les deux valeurs finiront par coïncider. En revanche, dès que le paramètre deviendra instable, la moyenne à court terme va suivre les variations du paramètre plus rapidement que la moyenne à long terme. La différence entre les deux moyennes ne sera plus nulle ou quasi-nulle, mais prendra une valeur positive (en cas d'augmentation du paramètre) ou négative (en cas de diminution), la valeur absolue de cet écart dépendant du paramètre analysé et de la vitesse de variation de celui-ci.

Pour opérer la classification, un ou plusieurs seuils sont fixés et chacun des paramètres PEA I ou PEA II (ou une combinaison de ces deux paramètres) est comparé à un seuil prédéterminé. Le résultat de la comparaison peut être combiné de diverses manières avec le résultat de comparaisons semblables d'autres paramètres (dont la fréquence cardiaque) pour produire un signal de sortie à deux ou plusieurs états, chaque état étant associé à une type particulier de syncope.

On donnera dans l'exemple plus bas des détails de mise en oeuvre d'une telle technique.

Il est également possible d'utiliser un processus de type "machine à états", où les résultats des comparaisons aux divers seuils sont appliqués à un système à transition d'états et à mémoire, qui opère la classification en fonction d'un schéma d'évolution plus complexe.

D'autres types d'analyses peuvent également être mises en oeuvre, par exemple des techniques de corrélation, d'analyse de morphologie du signal, d'analyse fréquentielle, d'analyse par ondelettes, etc.

La classification peut également prendre en compte non seulement les paramètres PEA I et/ou PEA II et la fréquence cardiaque, mais encore des signaux délivrés par un capteur d'activité, un capteur de ventilation-minute, etc.

*Exemple*

On va maintenant décrire un exemple d'algorithme de classification basé sur l'analyse combinée du premier pic d'accélération (PEA I), du second pic d'accélération (PEA II) et de la fréquence cardiaque.

Ces trois quantités sont mesurées à chaque cycle cardiaque et un algorithme calcule, pour chacune d'entre elles, deux moyennes glissantes, à long terme et à court terme, ces moyennes étant mises à jour régulièrement (à chaque cycle, tous les quatre cycles, ou tous les dix cycles, etc.).

L'algorithme détermine ainsi les six quantités suivantes :

$PEA1_{LT}$ :     moyenne glissante à long terme (par exemple sur 1000 cycles) du paramètre PEA I,
$PEA1_{CT}$ :     moyenne glissante à court terme (par exemple sur 30 cycles) du paramètre PEA I,
$PEA2_{LT}$ :     moyenne glissante à long terme (par exemple sur 1000 cycles) du paramètre PEA II,
$PEA2_{CT}$ :     moyenne glissante à court terme (par exemple sur 30 cycles) du paramètre PEA II,
$FC_{LT}$     moyenne glissante à long terme (par exemple sur 5000 cycles) de la fréquence cardiaque,
$FC_{CT}$ :     moyenne glissante à court terme (par exemple sur 100 cycles) de la fréquence cardiaque.

Pour classifier une syncope, l'algorithme évalue les trois quantités booléennes suivantes :

$$( PEA1_{CT} < k_1.PEA1_{LT} ) \ \& \ ( FC_{CT} > k_2.FC_{LT} ) \tag{1}$$

$$( PEA2_{CT} < k_3.PEA2_{LT} ) \ \& \ ( FC_{CT} > k_4.FC_{LT} ) \tag{2}$$

$$( PEA2_{CT} < k_5.PEA2_{LT} ) \ \& \ ( FC_{CT} > k_6.FC_{LT} ) \tag{3}$$

Un exemple (bien entendu non limitatif) d'application numérique pour les facteurs $k_1$ à $k_6$ est :

$$( PEA1_{CT} < 0,75.PEA1_{LT} ) \ \& \ ( FC_{CT} > 1,15.FC_{LT} ) \tag{1}$$

$$( PEA2_{CT} < 0,95.PEA2_{LT} ) \ \& \ ( FC_{CT} > 1,15.FC_{LT} ) \tag{2}$$

$$( PEA2_{CT} < 0,55.PEA2_{LT} ) \ \& \ ( FC_{CT} > FC_{LT} ) \tag{3}$$

La condition (1), si elle est vérifiée, indique que bien que la fréquence cardiaque augmente au-dessus de sa valeur de base, le paramètre PEA I décroît - c'est-à-dire que la contractilité myocardique diminue, révélant une baisse d'activité du système sympathique.

La condition (2), si elle est vérifiée, indique que bien que la fréquence cardiaque augmente au-dessus de la valeur de base, le paramètre PEA II diminue - c'est-à-dire que la pression sanguine diastolique périphérique décroît.

La condition (3), qui correspond à la condition (2) mais avec des critères plus stricts, révèle que le système nerveux autonome n'est plus en mesure de réguler la stabilité de la pression sanguine.

À chacune de ces conditions est associé un type particulier de syncope :

- syncope de type I pour la condition (1), c'est-à-dire une diminution du PEA I précédant la survenue de la syncope, associée à une fréquence cardiaque située au-dessus du niveau de base : l'observation de ce type de syncope montre clairement l'influence du système nerveux autonome sur l'apparition des symptômes.
- syncope de type II pour la condition (2), où la diminution du PEA II associée à une fréquence cardiaque située au-dessus du niveau de base révèle au contraire une probable étiologie vaso-dépressive.
- syncope de type III pour la condition (3), avec un déclin rapide du PEA II pas ou peu corrélé à une augmentation de la fréquence cardiaque, qui révèle l'influence dominante du système nerveux autonome sur l'apparition de la syncope.

Comme on peut le voir, la mise en oeuvre de l'appareillage de l'invention est particulièrement simple et non invasive, dans la mesure où il suffit d'appliquer le capteur accélérométrique sur la cage thoracique du patient.

De plus, la mise en oeuvre de cet appareillage n'allonge pas la durée du diagnostic, puisqu'il est mis en oeuvre pendant la session de l'épreuve d'inclinaison qui, de toute façon, aurait été réalisée.

De façon originale, l'appareillage de l'invention analyse le comportement du système nerveux autonome dans la période précédant la syncope vasovagale, approche qui jusqu'à présent n'avait guère été explorée, car il était estimé qu'elle requérait des procédures complexes et longues à mettre en oeuvre, ou bien était limitée à certains types de patients appareillés d'un dispositif implanté approprié (comme dans l'article précité de Mangin).

Diverses mises en oeuvre peuvent être envisagées en variante ou en complément du mode de classification ci-dessus, donné en exemple.

Il est notamment possible de classifier la syncope à partir d'une analyse de l'énergie contenue dans le signal d'accélération endocardiaque au niveau du pic PEA I et/ou du pic PEA II, ou encore syncope à partir d'une analyse de ce même signal d'accélération endocardiaque, telle qu'une analyse temps/fréquence ou une analyse de l'aire sous la courbe de ce signal, ou encore une analyse de la largeur du pic.

**Revendications**

1. Un appareillage pour le diagnostic non invasif des états de syncope vasovagale chez un patient (12) placé sur une table basculante (10) et soumis à une épreuve d'inclinaison, cet appareillage comportant :

   - des moyens de recueil de l'accélération endocardiaque du patient,
   - des moyens de recueil de la fréquence cardiaque du patient, et
   - des moyens d'analyse (18, 20), recevant en entrée ladite accélération endocardiaque et ladite fréquence cardiaque et délivrant en sortie une information sur l'activité sympathico-vagale du patient,

   appareillage **caractérisé en ce que** :

   - les moyens de recueil de l'accélération endocardiaque comprennent un capteur accélérométrique externe (14) apte à être maintenu en contact avec la paroi thoracique du patient, et
   - les moyens d'analyse comprennent des moyens classificateurs aptes, en cas de survenue d'une syncope, à déterminer un type de syncope parmi plusieurs, en fonction des valeurs de l'accélération endocardiaque et de la fréquence cardiaque recueillies au cours d'une pluralité de cycles cardiaques précédant la survenue de la syncope.

2. L'appareillage de la revendication 1, dans lequel :

   - les moyens d'analyse sont des moyens aptes à déterminer au moins une valeur fonction de l'un et/ou de l'autre de deux pics de l'accélération endocardiaque au cours d'un cycle donné, ces deux pics comprenant un premier pic (PEA I) lors de la phase de contraction ventriculaire isovolumique et un second pic (PEA II) lors de la phase de relaxation ventriculaire isovolumique, et
   - les moyens classificateurs sont des moyens aptes à déterminer le type de syncope en fonction de valeurs de pic(s) d'accélération endocardiaque et de la fréquence cardiaque recueillies au cours d'une pluralité de cycles cardiaques précédant la survenue de la syncope.

3. L'appareillage de la revendication 2, dans lequel les moyens classificateurs sont des moyens aptes à déterminer le type de syncope en fonction d'une moyenne des valeurs de pic(s) d'accélération endocardiaque recueillies au cours d'une pluralité de cycles cardiaques précédant la survenue de la syncope.

4. L'appareillage de la revendication 2, dans lequel les moyens classificateurs sont des moyens aptes à déterminer le type de syncope en fonction de la variation des valeurs de pic(s) d'accélération endocardiaque recueillies au cours d'une pluralité de cycles cardiaques précédant la survenue de la syncope.

5. L'appareillage de la revendication 2, dans lequel les moyens d'analyse comprennent des moyens pour déterminer :

   - une moyenne à long terme ($PEA1_{LT}$) et une moyenne à court terme ($PEA1_{CT}$) des valeurs des premiers pics d'accélération endocardiaque (PEA I) relevées lors de la phase de contraction ventriculaire isovolumique au cours d'une pluralité de cycles successifs, et/ou
   - une moyenne à long terme ($PEA2_{LT}$) et une moyenne à court terme ($PEA2_{CT}$) des valeurs des seconds pics d'accélération endocardiaque (PELA II) relevées lors de la phase de relaxation ventriculaire isovolumique au cours d'une pluralité de cycles successifs, et
   - une moyenne à long terme ($FC_{LT}$) et une moyenne à court terme ($FC_{CT}$) des valeurs de fréquence cardiaque,

et dans lequel les moyens classificateurs sont des moyens aptes à déterminer le type de syncope en fonction de différences entre les moyennes à long terme et à court terme des valeurs de pic(s) d'accélération endocardiaque recueillies au cours d'une pluralité de cycles cardiaques précédant la survenue de la syncope.

6. L'appareillage de la revendication 5, dans lequel les moyens classificateurs sont des moyens aptes à déterminer un premier type de syncope lorsque :

- à un premier facteur de proportionnalité ($k_1$) près, la moyenne à court terme des valeurs des premiers pics d'accélération ($PEA1_{CT}$) est inférieure à la moyenne à long terme des valeurs des premiers pics d'accélération ($PEA1_{LT}$), et
- à un deuxième facteur de proportionnalité ($k_2$) près, la moyenne à court terme des valeurs de fréquence cardiaque ($FC_{CT}$) est supérieure à la moyenne à long terme des valeurs de fréquence cardiaque ($FC_{LT}$).

7. L'appareillage de la revendication 5, dans lequel les moyens classificateurs sont des moyens aptes à déterminer un deuxième type de syncope lorsque :

- à un troisième facteur de proportionnalité ($k_3$) près, la moyenne à court terme des valeurs des seconds pics d'accélération ($PEA2_{CT}$) est inférieure à la moyenne à long terme des valeurs des seconds pics d'accélération ($PEA2_{LT}$), et
- à un quatrième facteur de proportionnalité ($k_4$) près, la moyenne à court terme des valeurs de fréquence cardiaque ($FC_{CT}$) est supérieure à la moyenne à long terme des valeurs de fréquence cardiaque ($FC_{LT}$)·

8. L'appareillage de la revendication 7, dans lequel les moyens classificateurs sont des moyens aptes à déterminer un troisième type de syncope lorsque:

- à un cinquième facteur de proportionnalité ($k_5$) près, inférieur audit troisième facteur de proportionnalité ($k_3$), la moyenne à court terme des valeurs des seconds pics d'accélération ($PEA2_{CT}$) est inférieure à la moyenne à long terme des valeurs des seconds pics d'accélération ($PEA2_{LT}$), et
- à un sixième facteur de proportionnalité ($k_5$) près, inférieur audit quatrième facteur de proportionnalité ($k_4$), la moyenne à court terme des valeurs de fréquence cardiaque ($FC_{CT}$) est supérieure à la moyenne à long terme des valeurs de fréquence cardiaque ($FC_{LT}$).

**Claims**

1. Apparatus for non-invasive diagnosis of states of vasovagal syncope in a patient placed on a tilt table and subjected to a tilt-test, the apparatus comprising:

· means for sensing the patient's endocardiac acceleration;
· means for sensing the patient's heart rate; and
· analyzer means receiving as inputs said endocardiac acceleration and said heart rate, and outputting information about the sympthetico-vagal activity of the patient;

wherein:

· the means for sensing endocardiac acceleration comprise an external accelerator sensor suitable for being held in contact with the patient's rib cage; and
· the analyzer means comprising classifier means suitable, in the event of a syncope occurring, for determining one type of syncope amongst a plurality of types as a function of the endocardiac acceleration and heart rate values sensed during a plurality of heart cycles preceding the occurrence of the syncope.

2. The apparatus of claim 1, in which:

· the analyzer means are means suitable for determining at least one value as a function of one and/or the other of two endocardiac acceleration peaks during a given cycle, these peaks comprising a first peak during the constant volume ventricular contraction stage and a second peak during the constant volume ventricular relaxation stage; and
· the classifier means are means suitable for determining the type of syncope as a function of the values of the endocardiac acceleration peak(s) and of the heart rate as sensed during a plurality of heart cycles preceding the occurrence of the syncope.

3. The apparatus of claim 2, in which the classifier means are means suitable for determining the type of syncope as a function of an average of the values of the endocardiac acceleration peak(s) sensed during a plurality of heart cycles preceding the occurrence of the syncope.

4. The apparatus of claim 2, in which the classifier means are means suitable for determining the type of syncope as a function of the variation in the values of the endocardiac acceleration peak(s) sensed during a plurality of heart cycles preceding the occurrence of the syncope.

5. The apparatus of claim 2, in which the analyzer means comprise means for determining:

· a long-term average and a short-term average of the values of the first endocardiac acceleration peaks sensed during the constant volume ventricular contraction stage during a plurality of successive cycles; and/or
· a long-term average and a short-term average of the values of the second endocardiac acceleration peaks sensed during the constant volume ventricular relaxation stage during a plurality of successive cycles; and
· a long-term average and a short-term average of the heart rate values;

and in which the classifier means are means suitable for determining the type of syncope as a function of differences between the long-term and short-term averages of the values of the endocardiac acceleration peak(s) sensed during a plurality of heart cycles preceding the occurrence of the syncope.

6. The apparatus of claim 5, in which the classifier means are means suitable for determining a first type of syncope when:

· the short-term average of the values of the first acceleration peaks is less than the long-term average of the values of the first acceleration peaks weighted by a first proportionality factor; and
· the short-term average of the heart rate values is greater than the long-term average of the heart rate values weighted by a second proportionality factor.

7. The apparatus of claim 5, in which the classifier means are means suitable for determining a second type of syncope when:

· the short-term average of the values of the second acceleration peaks is less than the long-term average of the values of the first acceleration peaks weighted by a third proportionality factor; and
· the short-term average of the heart rate values is greater than the long-term average of the heart rate values weighted by a fourth proportionality factor.

8. The apparatus of claim 7, in which the classifier means are means suitable for determining a third type of syncope when:

· the short-term average of the values of the second acceleration peaks is less than the long-term average of the values of the first acceleration peaks weighted by a fifth proportionality factor, less than said third proportionality factor; and
· the short-term average of the heart rate values is greater than the long-term average of the heart rate values weighted by a sixth proportionality factor, less than said fourth proportionality factor.

**Patentansprüche**

1. Diagnosevorrichtung zur nichtinvasiven Diagnose von Zuständen einer vaso-vagalen Synkope bei einem auf einen Kipptisch (10) gelegten und einem Kipptischtest ausgesetzten Patienten (10), wobei genannte Vorrichtung

- Mittel zur Erfassung der endokardialen Beschleunigung des Patienten,
- Mittel zur Erfassung der Herzfrequenz des Patienten, und
- Analysemittel (18, 20), die am Eingang genannte endokardiale Beschleunigung und genannte Herzfrequenz empfangen und am Ausgang eine Information über die sympathiko-vagale Aktivität des Patienten liefern,

umfasst,
**dadurch** gekennzeichent, dass :

- die Mittel zur Erfassung der endokardialen Beschleunigung einen externen Beschleugingungssensor (14) umfassen, der geeignet ist, in Kontakt mit der Brustwand des Patienten gehalten zu sein, und
- die Analysemittel Klassifizierungsmittel umfassen, die geeignet sind, beim Auftreten einer Synkope zwischen mehreren Typen den Typ der Synkope in Abhängigkeit von den während einer Vielzahl von dem Auftreten vorangehenden Herzzyklen erfassten Werten der endokardialen Beschleunigung und der Herzfrequenz zu ermitteln.

2. Die Vorrichtung gemäss Anspruch 1, in welcher

- die Analysemittel Mittel sind, die geeignet sind wenigstens einen Wert zu ermitteln, der von einem oder dem anderen der zwei endokardialen Beschleunigungsspitzen während einem gegebenen Zyklus abhängt, wobei genannte Spitzen eine erste Spitze (PEA I) während der isovolumischen Kammerkontraktionsphase und eine zweite Spitze (PEA II) während der isovolumischen Kammerrelaxationsphase umfassen, und
- die Klassifierungsmittel Mittel sind, die geeignet sind, beim Auftreten einer Synkope zwischen mehreren Typen den Typ der Synkope in Abhängigkeit von während einer Vielzahl von dem Auftreten vorangehenden Herzzyklen erfassten Werten der endokardialen Beschleunigungs-spitze(n) und der Herzfrequenz zu ermitteln.

3. Die Vorrichtung gemäss Anspruch 2, in welcher die Klassifierungsmittel Mittel sind, die geeignet sind, den Typ der Synkope in Abhängigkeit von einem Mittelwert der während einer Vielzahl von dem Auftreten vorangehenden Herzzyklen erfassten Werten der endokardialen Beschleunigungsspitze(n) zu ermitteln.

4. Die Vorrichtung gemäss Anspruch 2, in welcher die Klassifierungsmittel Mittel sind, die geeignet sind, den Typ der Synkope in Abhängigkeit von der Variation der während einer Vielzahl von dem Auftreten vorangehenden Herzzyklen erfassten Werte der endokardialen Beschleunigungsspitze(n) zu ermitteln.

5. Die Vorrichtung gemäss Anspruch 2, in welcher die Analysemittel Mittel umfassen zur Ermittlung :

- eines langfristigen Mittelwerts ($PEA1_{LT}$) und eines kurzfristigen Mittelwerts ($PEA1_{CT}$) der während der isovolumischen Kammerkontraktionsphase während einer Vielzahl von aufeinanderfolgenden Zyklen erfassten Werte der ersten endokardialen Beschleunigungsspitzen (PEA I), und/oder
- eines langfristigen Mittelwerts ($PEA2_{LT}$) und eines kurzfristigen Mittelwerts ($PEA2_{CT}$) der während der isovolumischen Kammerkontraktionsphase während einer Vielzahl von aufeinanderfolgenden Zyklen erfassten Werte der zweiten endokardialen Beschleunigungsspitzen (PEA II), und
- eines langfristigen Mittelwerts ($FC_{LT}$) und eines kurzfristigen Mittelwerts ($FC_{CT}$) der Herzfrequenzwerte,

und in welcher die Klassifierungsmittel Mittel sind, die geeignet sind, den Typ der Synkope in Abhängigkeit von Unterschieden zwischen den lang- und kurzfristigen Mittelwerten der während einer Vielzahl von dem Auftreten vorangehenden Herzzyklen erfassten Werten der endokardialen Beschleunigungsspitze(n) zu ermitteln.

6. Die Vorrichtung gemäss Anspruch 5, in welcher die Klassifierungsmittel Mittel sind, die geeignet sind, einen ersten Synkopetyp zu bestimmen, wenn :

- bis auf einem ersten Proportionalitätsfator ($k_1$) der kurzfristige Mittelwert der Werte der ersten Beschleunigungsspitzen ($PEA1_{CT}$) kleiner ist als der langfristige Mittelwert der Werte der ersten Beschleunigungswerte ($PEA1_{LT}$), und
- bis auf einem zweiten Proportionalitätsfator ($k_2$) der kurzfristige Mittelwert der Werte der Herzfrequenz ($FC_{CT}$) grösser ist als der langfristige Mittelwert der Werte der Herzfrequenz ($FC_{LT}$).

7. Die Vorrichtung gemäss Anspruch 5, in welcher die Klassifierungsmittel Mittel sind, die geeignet sind, einen zweiten Synkopetyp zu bestimmen, wenn :

- bis auf einem dritten Proportionalitätsfator ($k_3$) der kurzfristige Mittelwert der Werte der zweiten Beschleunigungsspitzen ($PEA2_{CT}$) kleiner ist als der langfristige Mittelwert der Werte der zweiten Beschleunigungswerte ($PEA2_{LT}$), und
- bis auf einem vierten Proportionalitätsfator ($k_4$) der kurzfristige Mittelwert der Werte der Herzfrequenz ($FC_{CT}$)

grösser ist als der langfristige Mittelwert der Werte der Herzfrequenz ($FC_{LT}$).

**8.** Die Vorrichtung gemäss Anspruch 7, in welcher die Klassifierungsmittel Mittel sind, die geeignet sind, einen dritten Synkopetyp zu bestimmen, wenn :

- bis auf einem fünften Proportionalitätsfator ($k_5$), der kleiner ist als genannter dritte Proportionalitäts-faktor ($k_3$), der kurzfristige Mittelwert der Werte der zweiten Beschleunigungsspitzen ($PEA2_{CT}$) kleiner ist als der langfristige Mittelwert der Werte der zweiten Beschleunigungswerte ($PEA1_{LT}$), und
- bis auf einem sechsten Proportionalitätsfator ($k_5$), der kleiner ist als genannter vierte Proportionalitäts-faktor ($k_4$), der kurzfristige Mittelwert der Werte der Herzfrequenz ($FC_{CT}$) grösser ist als der langfristige Mittelwert der Werte der Herzfrequenz ($FC_{LT}$) .

FIG_1

FIG_2

FIG_3

FIG_4

## RÉFÉRENCES CITÉES DANS LA DESCRIPTION

**Documents brevets cités dans la description**

- US 20040215263 A
- US 5957957 A
- EP 0515319 A
- EP 0655260 A

**Littérature non-brevet citée dans la description**

- **Mangin.** Simultaneous Analysis of Heart Rate Variability and Myocardial Contractility During Head-Up Tilt in Patients with Vasovagal Syncope. *Journal of Cardiovascular Electro-physiology,* Juin 2001, vol. 12 (6), 639-644